# EUROPEAN PATENT SPECIFICATION

(11) **EP 0 561 427 B1**
(45) Date of publication and mention of the grant of the patent: **16.07.1997**
(21) Application number: 93108007.1
(22) Date of filing: 02.04.1984
(51) Int. Cl.: C07K 16/36, C12N 5/20

(54) **Monoclonal antibodies to new factor VIII coagulant polypeptides**
Monoklonale Antikörper gegen neue Faktor-VIII Gerinnungspolypeptide
Anticorps monoclonaux dirigés contre des nouveaux polypeptides coagulants de facteur VIII

(30) Priority: 31.03.1983 US 481105; 30.11.1983 US 556508
(43) Date of publication of application: 22.09.1993
(62) Divisional of application: 84103630.4
(73) Proprietor: THE SCRIPPS RESEARCH INSTITUTE, La Jolla, CA 92037 (US)
(72) Inventor: Zimmerman, Theodore S., La Jolla, CA (US); Fulcher, Carol A., La Jolla, CA 92037 (US)
(74) Representative: VOSSIUS & PARTNER

(56) References cited:
- BLOOD vol. 59, no. 3, March 1982, NEW YORK, N.Y., US pages 594 - 600 D.N. FASS ET AL. 'MONOCLONAL ANTIBODIES TO PORCINE FACTOR VIII COAGULANT AND THEIR USE IN THE ISOLATION OF ACTIVE COAGULANT PROTEIN.'
- PROCEEDINGS OF THE NATIONAL ACADEMY OF SCIENCES OF USA. vol. 79, no. 5, March 1982, WASHINGTON US pages 1648 - 1652 C.A. FULCHER ET AL. 'CHARACTERIZATION OF THE HUMAN FACTOR VIII PROCOAGULANT PROTEIN WITH A HETEROLOGOUS PRECIPITATING ANTIBODY.'
- BLOOD vol. 61, no. 4, April 1983, NEW YORK, N.Y., US pages 807 - 811 C.A. FULCHER ET AL. 'THROMBIN PROTEOLYSIS OF PURIFIED FACTOR VIII PROCOAGULANT PROTEIN: CORRELATION OF ACTIVATION WITH GENERATION OF A SPECIFIC POLYPEPTIDE.'

## Description

This invention relates to a monoclonal antibody of class IgG produced by hybridoma formed by fusion of cells from a mouse myeloma and spleen cells from a mouse previously immunized with human factor VIII:C, which antibody reacts with human factor VIII:C polypeptides. The invention has utility in the therapy for classic hemophilia, and in the further study and characterization of the polypeptide or polypeptide complexes which provide desired clotting behavior to the blood of humans and other mammals.

It has long been known that plasma factor VIII plays a crucial role in blood coagulation, and that thrombin activates the coagulant effect of factor VIII. Recent attempts to characterize factor VIII have postulated that factor VIII is a complex of at least two polypeptides, which are known as VIII:C and VIII:R, and have found coagulant activity to reside in the VIII:C portion. Studies of the effect of thrombin on factor VIII:C have led to the conclusion that thrombin activates this factor by breaking it down into several smaller polypeptides. However, no prior studies have been able to associate thrombin-induced factor VIII activation in humans with any defined polypeptides formed from human factor VIII:C.

For instance, Hoyer and Trabold, in "The effect of thrombin on human factor VIII", J. Lab. Clin. Med., 97:50-64 (1981), sought to purify human factor VIII:C by immunoadsorbent chromatography using a polyclonal antibody to factor VIII:R raised in the rabbit. They then incubated the factor VIII:C with purified human α-thrombin, and determined that small amounts of the thrombin activated the factor VIII:C whereas larger amounts activated it less or not at all. They also concluded that thrombin activation is accompanied by a decrease in the size of the protein, and they proposed a molecular weight of about 116,000 for the activated factor VIII:C. Most significantly, they could not identify specific polypeptides that retained factor VIII:C activity and VIII:C antigen determinants.

Fulcher, C.A. and Zimmerman, T.S. in "Characterization of the human factor VIII procoagulant protein with a heterologous precipitating antibody", Proc. Natl. Acad. of Sci. USA, 79:1648-1652 (1982) obtained highly purified human factor VIII:C from plasma concentrate by passing the concentrate through a column containing a monoclonal antibody to factor VIII:R, eluting the VIII:C from the adsorbed VIII:C/VIII:R complex, and concentrating the factor VIII:C on a second column. The purified factor VIII:C was then analyzed by sodium dodecyl sulfate/polyacrylamide gel electrophoresis (hereafter, "SDS-PAGE"), both before and after addition of thrombin to the purified material. The purified factor VIII:C prior to thrombin addition showed a wide array of bands on SDS-PAGE corresponding to polypeptides of various molecular weights (Mᵣ), including a relatively strong doublet at Mᵣ of 80,000 and 79,000, and at least six additional faintly staining polypeptides with larger Mᵣ including one at Mᵣ about 92,000. Addition of thrombin to the purified factor VIII:C caused the diminution or disappearance of all of the polypeptides shown prior to thrombin addition.

The coagulant activity of the plasma concentrate rose following thrombin addition to a maximum of three times that of the material prior to thrombin addition, and then diminished. The coagulant activity of the purified factor VIII:C also rose to a maximum of three times that of the pre-activated material. That is, the thrombin had essentially the same activating effect on the factor VIII:C in each case. Thus, whereas the purified factor VIII:C possessed a reported specific activity some 3280 times that of the starting material, one skilled in this art would conclude that the increase in specific activity was due to the high degree of purification achieved. There is no basis in this article for ascribing activated coagulant activity to any specific one or more of the large number of polypeptides associated with the bands observed in the purified factor VIII:C prior to activation with thrombin.

The present invention relates to a monoclonal antibody which binds to human Factor VIII:C and exhibits one of the following binding profiles with polypeptides derived from human Factor VIII:C:
(A) binding to polypeptides derived from human Factor VIII:C which have a molecular weight of 92,000, ≥ 108,000 and 44,000 but not binding to polypeptides derived from human Factor VIII:C which exhibit a doublet of Mᵣ = 79,000-80,000 or those which exhibit a doublet of Mᵣ = 71,000-72,000,
(B) binding to polypeptides derived from human Factor VIII:C which have a molecular weight of 92,000, ≥ 108,000 and 54,000, but not binding to polypeptides derived from human Factor VIII:C which exhibit a doublet of Mᵣ = 79,000-80,000 or those which exhibit a doublet of Mᵣ = 71,000-72,000;
(C) binding to polypeptides derived from human Factor VIII:C which exhibit a doublet of Mᵣ = 79,000-80,000 and to ≥ 108,000 polypeptides but not binding to polypeptides derived from human Factor VIII:C which have a molecular weight of 44,000, 54,000, or 92,000 and to polypeptides which exhibit a doublet of Mᵣ = 71,000-72,000;
(D) binding to polypeptides derived from human Factor VIII:C which have a molecular weight of ≥ 108,000 but not binding to polypeptides derived from human Factor VIII:C which have a molecular weight of less than 108,000;
wherein the molecular weights are determined by subjecting the polypeptides to an SDS-PAGE under reducing conditions.

Other aspects of the invention include biological preparations containing antibodies exhibiting different reaction profiles, hybridomas secreting such antibodies and an immunoadsorbent column to which such antibodies are bound.

The polypeptide coagulants exhibiting factor VIII:C coagulant activity and factor VIII:C immunological behavior, and showing characteristic Mᵣ value(s) when analyzed by sodium dodecyl sulfate-polyacrylamide gel electrophoresis as well as the preparation and characterization thereof are described in the parent application EP 84 10 3630.4 (EP-A 0 123 945).

In the process of the preparation of the coagulant, the desired complex can also be concentrated and/or recovered by passing the reaction mixture, which can already have been concentrated by another technique, through an immunoadsorbent column containing anti-human VIII:C antibodies, according to the present invention or equivalent antibodies against VIII:C other than human, that react with the polypeptide(s) of the complex. The antibodies are attached to agarose (see Example I below). Several antibodies which can be used to concentrate the complex and/or isolate certain of the polypeptides thereof are described below. The active VIII:C complex adsorbs preferentially to the column, and is then eluted from the column with a solution of calcium ions (e.g., CaCl₂) which can optionally also contain a non-ionic surfactant. Suitable non-ionic surfactants include alkyl phenyl polyoxyethylenes such as Triton-X-100®, -N-101®, or -X-405® (Eastman Chemical Co.); Tween-20®, -60® or -80® (Sigma Chemical Co.); and Nonidet P-40® (Sigma Chemical Co.); all of these are well-known articles of commerce having known chemical formulas.

The amounts of calcium ion and surfactant to use should be high enough to desorb the polypeptide complex, but not so high that the eluant inactivates the polypeptide. A calcium ion concentration of up to 0.5 M or even up to 1.0 M is satisfactory, and 0.25 M is preferred. A surfactant concentration of up to 1 wt. % is satisfactory, and about 0.1 wt. % is preferred. The eluant is applied to the immunoadsorbent column at 1 to 8 bed volumes per hour, and preferably about 3 to 4 per hour. Too high a flow rate risks disruption of the column and non-absorption of the polypeptide complex. The skilled practitioner will readily adapt these guidelines to immunoadsorbent processes other than fixed-bed columns.

### EXAMPLE I

This Example shows how factor VIII:C was purified from commercial concentrate and digested with purified α-thrombin. A monoclonal antibody to factor VIII:C was produced and used to identify VIII:C polypeptides. At several selected points during the digestion, portions of the digestion mixture were assayed for VIII:C (coagulant) activity, and for protein bands using SDS-PAGE.

Purification of VIII:C. All steps were at room temperature. Chemicals were reagent grade. 40 bottles of commercial factor VIII concentrate (provided by Armour Pharmaceutical) were reconstituted in 1000 ml of VIII:C buffer (0.02 M imidazole/0.15M sodium chloride/0.1 M L-lysine HCl/0.02% sodium azide, pH 6.8). This sample, which contained a total of 17,000 units of VIII:C activity, was applied to a 2.5 - 3.0 liter bed volume immunoadsorbent column. The column was cyanogen bromide-activated agarose (Sepharose 4B, Pharmacia, Piscataway, New Jersey), to which monoclonal antibodies to VIII:R had been covalently bonded. The antibodies were raised and attached to the column as described in the aforementioned U.S. Patent No. 4,361,509. The antibodies were precipitated from ascites fluid using 50% ammonium sulfate, reprecipitated two more times, and then attached to the column at a density of 2-4 mg/ml of Sepharose. The immunoadsorbent was pre-eluted with 3M sodium thiocyanate, washed with VIII:C buffer (0.02M imidazole HCl pH 7.0, 0.15 M NaCl, 0.1 M L-lysine-HCl, 0.02% sodium azide), treated twice with 2mM di-isopropyl fluorophosphate, and then the concentrate was added.

The column was washed with 20 liters of VIII:C buffer containing 0.15 M sodium chloride, and VIII:C was then eluted from the VIII:R with VIII:C buffer containing 0.35 M calcium chloride. Active fractions were pooled and concentrated under nitrogen pressure 100-fold in an Amicon stirred cell with a YM10 membrane. The concentrate was then diluted 1:10 in VIII:C buffer and applied to a 4 ml column of aminohexyl-Sepharose (Pharmacia) equilibrated in VIII:C buffer containing 0.025 M calcium chloride, VIII:C was eluted in high concentration with VIII:C buffer containing 0.3 M calcium chloride at a flow rate of 10 ml/hr. The concentrated immunoadsorbent pool was adjusted to 0.25 M calcium chloride and adsorbed twice for 1 h each time with 1/10 vol/vol of a mixture of monoclonal anti-fibrinogen, anti-fibronectin and anti-vWF antibodies which had been coupled to cyanogen bromide-activated Sepharose.

Production of monoclonal antibody against VIII:C. Monoclonal antibodies were produced as described in U.S. Patent No. 4,361,509 using purified VIII:C as immunogen. The antibodies were selected with a solid-phase assay in Linbro-Titertek® (Flow Laboratories, Inglewood, CA) plates and an enzyme-linked immunoadsorbent (ELISA) detection system described in Engvall, E. and Perlmann, P. "Enzyme-linked immunoadsorbent assay (ELISA), Quantitative assay of immunoglobulin G" Immunochemistry 8:871-874 (1971) using a peroxidase-antibody conjugate (Zymed Laboratories, Burlingame, CA). The plates were coated with 100 ng of purified VIII:C per well. The ELISA-positive culture supernatant of the clone selected for use in this study also inhibited plasma VIII:C activity.

Thrombin activation time course analysis of purified VIII:C. Purified human α-thrombin(sp. act. 2534 U/mg, final concentration 0.5 U/ml), was added to the purified VIII:C (final concentration 167 µg/ml) in imidazole saline buffer containing 0.04 M CaCl₂. Buffer alone was added to a control aliquot. The solutions were incubated at room temperature and at various time intervals samples of the VIII:C-thrombin mixture were added to tubes containing p-APMSF (California Medicinal Chemistry Co.) to inactivate the thrombin rapidly and irreversibly. In order to minimize hydrolysis of p-APMSF, it was diluted 1:10 from a stock solution (100 mM in methanol) into imidazole saline buffer 60 seconds before reaction with the VIII:C-thrombin samples. The final p-APMSF concentration was 1 mM. The control aliquot was treated similarly with p-APMSF at the start of the experiment. At the end of the 60 minute time course, all VIII:C samples were assayed for VIII:C activity using an activated partial thromboplastin time assay described in the literature and then prepared for SDS-PAGE as described in the parent application.

Results. The specific activity of the purified factor VIII:C was 2000 units/mg. Thrombin activation of purified VIII:C activity was analyzed over a 60 minute time course. Before thrombin exposure the untreated VIII:C sample showed the characteristic array of VIII:C forms ranging from a doublet at Mᵣ = 79,000-80,000 to a band at Mᵣ = 188,000. A band above Mᵣ = 188,000 and two bands below Mᵣ = 79,000 did not bind to the monoclonal anti-VIII:C antibody immunoadsorbent. The bands between Mᵣ=79,000 and Mᵣ=188,000 did bind to the anti-VIII:C antibody.

During the first 5 minutes of the thrombin activation time course, all but one of the monoclonal anti-VIII:C antibody reactive bands with an Mᵣ greater than 92,000 gradually disappeared and were undetectable when VIII:C activity reached its peak at 5 minutes.

A band at Mᵣ = 122,000 appeared thrombin-resistant in only some experiments, but after extensive thrombin treatment neither this band nor any other band was reactive with the immobilized monoclonal anti-VIII:C. antibody.

A band at Mᵣ = 92,000 increased in intensity as VIII:C activity increased. A Mᵣ = 79,000-80,000 doublet appeared to be converted to a doublet at Mᵣ = 71,000-72,000, with the latter form predominant from 5 to 60 min. as VIII:C activity decreased. Two bands, at Mᵣ = 54,000 and Mᵣ = 44,000, became clearly visible from 5 to 60 min. The Mᵣ = 44,000 band also appeared as a doublet in some experiments. The Mᵣ = 71,000-72,000 doublet, the Mᵣ = 54,000 band and the Mᵣ = 44,000 band were not removed to a significant degree by the immobilized monoclonal anti-VIII:C antibody.

Scanning and integration of the gel discussed above allowed correlation of changes in polypeptide concentration with changes in VIII:C activity. The results are shown in the Table of the parent application. As shown therein, the Mᵣ = 92,000 band increased and then decreased in concentration in parallel with VIII:C activity. This suggests that the Mᵣ = 92,000 band is an active form of VIII:C whose concentration increased with thrombin activation. The Mᵣ = 54,000 and Mᵣ = 44,000 bands increased steadily in concentration between 1 and 40 min., even after activity of the mixture declined.

Most of the Mᵣ = 79,000-80,000 doublet was lost during the first 0.1 to 10 min. as VIII:C activity peaked, while most of the Mᵣ = 71,000-72,000 doublet appeared during this time and predominated even as VIII:C activity decreased. These data suggest that the Mᵣ = 71,000-72,000 doublet was derived from the Mᵣ = 79,000-80,000 doublet and that the Mᵣ = 71,000-72,000 doublet is inactive by itself. These data are consistant with the retention by the Mᵣ = 71,000-72,000 doublet of the ability to complex with the Mᵣ = 92,000 polypeptide; this complex would also have activity.

Direct evidence that the Mᵣ = 92,000 polypeptide is complexed with the Mᵣ = 79,000-80,000 doublet derives from experiments utilizing the anti-VIII:C monoclonal immunoadsorbent. It was first shown that the monoclonal antibody reacts predominantly with the Mᵣ = 79,000-80,000 doublet and not with the Mᵣ = 92,000 polypeptide. This was shown by electrophoretic transfer experiments. Then it was shown that the monoclonal anti-immunoadsorbent removed both the Mᵣ = 79,000-80,000 doublet and the Mᵣ=92,000 polypeptide from the solution. The Mᵣ=92,000 polypeptide could be eluted from the anti-VIII:C immunoadsorbent column with 10 mM EDTA, whereas the Mᵣ =79,000-80,000 doublet remained bound. The doublet could be subsequently eluted with 3M sodium thiocyanate. These experiments demonstrated that the Mᵣ=92,000 polypeptide bound to the immunoadsorbent because it was complexed with the Mᵣ=79,000-80,000 doublet and that it was this doublet which bound directly to the immunoadsorbent.

The present invention relates to monoclonal antibodies which are specific in an unforeseeable way to the various polypeptides which are formed by the reaction of factor VIII:C with a protease such as alpha-thrombin. Each antibody reacts with human factor VIII:C which has not been activated nor digested with thrombin or equivalent proteases. The antibodies are further characterized by their individual properties, as follows:
(A) One reacts with the Mᵣ =92,000 polypeptide described herein, with polypeptides of Mᵣ=108,000 and larger, and with the polypeptide of Mᵣ = 44,000 which is present in terminal-thrombin digests. It does not react with the polypeptide described herein which exhibits a doublet of Mᵣ = 79,000-80,000, nor with the polypeptide described herein which exhibits a doublet of Mᵣ = 71,000-72,000.
(B) One reacts with the Mᵣ=92,000 polypeptide described herein, with polypeptide-s of Mᵣ=108,000 and larger, and with the polypeptide of Mᵣ = 54,000 which is present in terminal thrombin digests. It does not react with the polypeptide described herein which exhibits a doublet of Mᵣ = 79,000-80,000, nor with the polypeptide described herein which exhibits a doublet of Mᵣ = 71,000-72,000.
(C) One reacts with the doublet of Mᵣ=79,000-80,000 and polypeptides of Mᵣ=108,000 and greater, but not with the polypeptide of Mᵣ=92,000, nor with the polypeptides of Mᵣ= 71,000-72,000, Mᵣ=54,000, nor Mᵣ=44,000.
(D) One reacts only with polypeptides of Mᵣ=108,000 and greater.

Each of these antibodies can be used to concentrate the complex described above, from mixtures which also contain other polypeptides. One such mixture is produced by partial digestion of human factor VIII:C with α-thrombin or an equivalent protease. Another such mixture is that produced by recombinant DNA techniques in which a desired polypeptide or complex is expressed by a microorganism and must be recovered from a mixture with other proteinaceous compounds. Antibody (A), (B), (C) or (D), or a combination of two, three, or all of them, can be attached to an immunoadsorbent column in the manner described in Example I, and a feed solution of the mixture containing the polypeptide(s) comprising the complex is poured through the column. Those polypeptides having Mᵣ of 92,000, 79,000-80,000, and 71,000-72,000 which are present in the feed solution adsorb onto the column, from which they can be eluted as taught hereinabove after the source solution has been washed through the column. The resulting eluted solution is thereby concentrated in the desired activated VIII:C complex compared to the feed solution.

The new antibodies are also useful for analytical purposes, to detect the occurrence of the reaction of human VIII:C with thrombin or other proteases, because of their ability to react with products of that reaction. An antibody having profile (B) and an antibody having profile (C) have been found which neutralize VIII:C coagulant activity when either is bound to factor VIII:C. This additional property can be of value in the diagnosis of hemophilia-related disorders.

The discovery of these antibodies also permits further characterization of the components of the polypeptide complex described herein. Thus, the polypeptide which exhibits a band of Mᵣ=92,000 contains (at least) two epitopes (i.e. antibody binding sites) that are not destroyed by thrombin digestion and are not present on the polypeptides which exhibit the doublet of Mᵣ=79,000-80,000 or the doublet of Mᵣ=71,000-72,000. One of these epitopes is also present on the polypeptide of Mᵣ=44,000, and the other on the polypeptide of Mᵣ=54,000. Thus, the Mᵣ =54,000 and Mᵣ=44,000 polypeptides derive from the Mᵣ=92,000 polypeptide. Also, the Mᵣ=92,000 and Mᵣ =79,000-80,000 polypeptides are derived from a common precursor or precursors. The discovery of antibodies having profile (B) and profile (C) each of which neutralizes factor VIII:C coagulant activity is further support that the Mᵣ=92,000 and Mᵣ=79,000-80,000 polypeptides are important to the coagulant function. The polypeptide exhibiting the doublet of Mᵣ=79,000-80,000 contains an epitope which is destroyed by thrombin digestion and which is not present on the polypeptide of Mᵣ =92,000.

These monoclonal antibodies can be prepared by the general steps of purification of human factor VIII:C; raising monoclonal antibodies to the purified VIII:C; partial digestion and activation of purified VIII:C to produce the polypeptide complex described above, including identification of the specific polypeptides; reaction of the anti-VIII:C antibodies with the products of the activation; and characterization of an antibody by identification of the polypeptide(s) with which it reacted. This sequence is set forth in more detail in Example II below. Alternatively, one can prepare an antibody by isolating the particular polypeptide of interest from the partial thrombin digestion products, for instance by immunoadsorbtion onto and then elution off of a column comprising agarose to which is coupled a monoclonal antibody known to react with the polypeptide of interest, and then raising a monoclonal antibody against that polypeptide using the procedure described in Example II .

### EXAMPLE II

Monoclonal antibodies to human Factor VIII:C were raised using the following procedure, starting from highly purified VIII:C which had been prepared by the process of U.S. Patent No. 4,361,509.

Mice were injected with highly purified factor VIII:C according to the following procedure. On day zero, the mice were injected intraperitoneally with a composition prepared by dissolving (or suspending) 10 µg of the protein in 0.1 ml of buffer containing 0.05 M Tris, 0.15 M sodium chloride, 0.02% sodium azide, 1 mM phenyl methyl sulfonyl fluoride, trasylol 10 units/ml at pH 7.3, and shaking with an equal volume of complete Freund's adjuvant. On day 14, the mice were again injected with the same material except that incomplete Freund's adjuvant was substituted for complete Freund's.adjuvant. On day 21, the injection of day 14 was repeated. On day 38, the mice were injected with purified VIII:C only. On day 42, the spleens of the mice were removed and fused according to a standard procedure, of the type described by J. P. Brown et al., "Protein Antigens of Normal and Malignant Human Cells Identified by Immunoprecipitation with Monoclonal Antibodies", JOURNAL OF BIOLOGICAL CHEMISTRY, Vol. 225, pp. 4980-4983 (1980). The standard technique was varied only to the extent that 35% polyethylene glycol 1000 was substituted for 50% polyethylene glycol.

The antibodies were selected using the assay procedure described above in Example I, in the paragraph with the heading "Production of monoclonal antibody against VIII:C", except that antibodies which did not neutralize VIII:C activity were also subcloned and treated as described below.

The clones which were positive were subcloned twice and stable clones producing antibody to VIII:C were then injected into the peritoneal cavities of Balb/C mice which had been pretreated intraperitoneally with 0.5 ml of pristane at least four days prior to injection of cells. Hybridoma cells were injected at concentrations of approximately 5 x 10⁶ cells per mouse in 0.5 ml of Dulbecco's modified Eagle's medium without fetal bovine serum. The mice were tapped when bloated and ascites fluid was collected in heparin at approximately 10 units/ml. Ascites fluid from multiple mice was pooled to provide a convenient volume for subsequent isolation of the monoclonal IgG. The antibodies were precipitated from ascites fluid using 50% ammonium sulfate, and then reprecipitated two more times. The anti-VIII:C antibodies so raised corresponding to (B), (C) and (D) above were bound to agarose beads and shown to bind purified VIII:C from solution.

Separate batches of VIII:C, one untreated and one subjected to thrombin proteolysis, were then analyzed by the SDS-PAGE steps described in the parent application. Then, each band was transferred electrophoretically (Western transfers) from the gel onto a nitrocellulose sheet. The apparatus used was Bio-Rad "Trans-Blot" cell, and Bio-Rad Model 160.1.6 power supply (Bio-Rad Laboratories, Richmond, California). The transfer buffer was 25 mM Tris with glycine added to pH 8.3, 20% methanol. Transfers were carried out over 16-24 hours at 90 volts and 100 milliamperes.

The specific polypeptides with which each of the antibodies reacted were determined using a procedure adapted from W. M. Burnette, "Western Blotting: Electrophoretic Transfer of Proteins from Sodium Dodecyl Sulfate Polyacrylamide Gels to Unmodified Nitrocellulose and Radiographic Detection with Antibodies and Radioiodinated Protein A", Analytical Biochemistry, Vol. 112, pp. 195-203 (1981).

"Buffer D" was 10 mM Tris chloride, 0.15 M NaCl, 0.02% sodium azide, pH 7.4. All reactions were carried out at room temperature.
1. Place the nitrocellulose sheet with the transferred protein in a tray containing 100 ml of Buffer D and 0.25% gelatin. Place the tray on a rotary shaker and shake slowly for 30 minutes.
2. Then add the monoclonal antibody to the tray (either 0.1% - 1% of ascites fluid or 1 mg of purified IgG). Shake for 120 minutes.
3. Wash the nitrocellulose sheet as follows:
   (a) 10 minutes with 100 ml of Buffer D.
   (b) 30 minutes with 100 ml of Buffer D and 0.05 % of Nonidet-P-40, with changes at 10 and 20 minutes.
   (c) 10 minutes with 100 ml of Buffer D.
4. Soak the nitrocellulose sheet in Buffer D plus 0.25% gelatin and I¹²⁵-labelled purified rabbit anti-mouse IgG for 30 minutes.
5. Wash the nitrocellulose sheet as follows:
   (a) 10 minutes with 100 ml of Buffer D.
   (b) 16-24 hours with 100 ml of Buffer D plus 0.1% Nonidet P-40 and 0.5 M NaCl.
   (c) 10 minutes with 100 ml of Buffer D.
6. Blot the nitrocellulose sheet between two sheets of filter paper and then store the nitrocellulose sheet in a sealed plastic bag.
7. To determine which antibodies and polypeptides had reacted,
   (a) Prepare an autoradiograph of the nitrocellulose sheet, using standard procedures known in the literature.
   (b) Compare the autoradiograph with a nitrocellulose sheet onto which VIII:C was transferred but which had been stained with Coomassie blue R250 rather than reacted with monoclonal antibody.

These steps demonstrated that the following distinct antibodies had been raised.

Four antibodies reacted with the polypeptide of Mᵣ=92,000, the Mᵣ =108,000 and larger polypeptides, one or the other of the polypeptides of Mᵣ=54,000 or 44,000 present in terminal thrombin digests, and no other polypeptides. This demonstrates the origin of these two latter polypeptides from the polypeptide of Mᵣ=92,000 as the result of thrombin cleavage. It also shows that two epitopes on the Mᵣ=92,000 polypeptide survive that cleavage, and that these epitopes are not present on the Mᵣ = 79,000-80,000 doublet.

A fifth antibody reacted with the doublet of Mᵣ = 79,000-80,000, and with polypeptides of Mᵣ=108,000 and greater, but not with the polypeptide of Mᵣ=92,000 nor with any of the polypeptides present in terminal thrombin digests. This demonstrates that the Mᵣ =79,000-80,000 doublet possesses an epitope which is not present on the Mᵣ =92,000 polypeptide; and that the epitope is destroyed by thrombin digestion of the Mᵣ = 79,000-80,000 doublet.

The reaction profiles of these five antibodies indicate that the Mᵣ =92,000 and Mᵣ = 79,000-80,000 polypeptides derive from a common precursor or precursors.

A sixth antibody reacted only with polypeptides of Mᵣ=108,000 and greater. Since it did not react with any polypeptides present in terminal thrombin digests, the epitope with which it reacted is destroyed by thrombin digestion.

To prepare and store a biological preparation of one or more of these antibodies, the corresponding monoclonal IgG may be isolated from heparinized pooled ascites fluid immediately after collection or a frozen portion of the stored solution may be thawed. Regardless of whether fresh or frozen material is used, the solution is brought to 4°C and treated with an equal volume of phosphate buffered saline solution (PBS) (PBS: 1.6 g sodium phosphate, monobasic monohydrate; 8.4 g g sodium phosphate, dibasic anhydrous; 61.4 g sodium chloride; water to 7 liters; pH=7.2). The diluted ascites is precipitated by dropwise addition with stirring at 4°C. Centrifugations are preferably carried out at 14,000 rpm for 60 minutes (30,000 x g). The supernatant solution of ascites is precipitated twice more with SAS and the mixture of precipitate and supernatant liquid stirred and centrifuged in the same manner as in the first cycle. The pellets resulting from the third precipitation are resuspended in a volume of P3S equal to that of the diluted ascites fluid and then dialyzed exhaustively against PBS. Clots appearing in the dialysis bags are removed by centrifugation at 20°C. The dialyzed IgG is adsorbed by stirring it with a 5% aqueous solution of aluminum hydroxide at room temperature and centrifuging at 20°C after adsorption. The adsorption treatment is repeated at least three more times using 2.5% aluminum hydroxide solution for each treatment after the first. The adsorbed IgG is brought to 4°C and reprecipitated once with SAS as described above. The precipitated pellets may be stored at -20°C until used.

Two preferred procedures for purifying the monoclonal antibodies and maintaining biological preparations containing them are described in P. L. Ey et al., "Isolation of Pure IgG₁, IgG₂ₐ, and IgG_{2b} Immunoglobulins from Mouse Serum Using Protein A-Sepharose." Immunochemistry, Vol. 15, pp. 429-436; and in C. Bruck et al., "One-Step Purification of Mouse Monoclonal Antibodies from Ascitic Fluid by DEAE Affi-Gel Blue Chromatography." J. Immunological Methods, Vol. 53, pp. 313-319 (1982).

## Claims (Claims for the following Contracting State(s): BE, CH, LI, DE, FR, GB, IT, LU, NL, SE)

1. A monoclonal antibody which binds to human Factor VIII:C and exhibits one of the following binding profiles with polypeptides derived from human Factor VIII:C:
(A) binding to polypeptides derived from human Factor VIII:C which have a molecular weight of 92,000, ≥ 108,000 and 44,000 but not binding to polypeptides derived from human Factor VIII:C which exhibit a doublet of Mᵣ = 79,000-80,000 or those which exhibit a doublet of Mᵣ = 71,000-72,000,
(B) binding to polypeptides derived from human Factor VIII:C which have a molecular weight of 92,000, ≥ 108,000 and 54,000, but not binding to polypeptides derived from human Factor VIII:C which exhibit a doublet of Mᵣ = 79,000-80,000 or those which exhibit a doublet of Mᵣ = 71,000-72,000;
(C) binding to polypeptides derived from human Factor VIII:C which exhibit a doublet of Mᵣ = 79,000-80,000 and to ≥ 108,000 polypeptides but not binding to polypeptides derived from human Factor VIII:C which have a molecular weight of 44,000, 54,000, or 92,000 and to polypeptides which exhibit a doublet of Mᵣ = 71,000-72,000;
(D) binding to polypeptides derived from human Factor VIII:C which have a molecular weight of ≥ 108,000 but not binding to polypeptides derived from human Factor VIII:C which have a molecular weight of less than 108,000;
wherein the molecular weights are determined by subjecting the polypeptides to an SDS-PAGE under reducing conditions.

2. The monoclonal antibody of claim 1 wherein the antibody is obtainable from a hybridoma formed by fusion of cells from a mouse myeloma and spleen cells from a mouse previously immunized with human Factor VIII:C, and the antibody is of the IgG class.

3. A monoclonal antibody according to any one of claims 1 or 2 having the binding profile (B) or (C) recited therein, further characterized in that it neutralizes the coagulant activity of human Factor VIII:C when said Factor VIII:C is bound to the antibody.

4. A biological preparation comprising an antibody exhibiting:
(a) binding profile A of claim 1 which is essentially free from antibodies exhibiting the other three of said profiles, or
(b) binding profile B of claim 1 which is essentially free from antibodies exhibiting the other three of said profiles, or
(c) binding profile C of claim 1 which is essentially free from antibodies exhibiting the other three of said profiles; or
(d) binding profile D of claim 1 which is essentially free from antibodies exhibiting the other three of said profiles.

5. A biological preparation comprising a mixture of antibodies according to any one of claims 1 or 2 which exhibit binding profiles A and B, which is essentially free of antibodies according to claim 1 or 2 which exhibit binding profiles C and D.

6. A biological preparation comprising a mixture of antibodies according to any one of claims 1 or 2 which exhibit binding profiles A, B, and C, which is essentially free of antibodies according to claim 1 or 2 which exhibit binding profile D.

7. A hybridoma that secretes a monoclonal antibody according to claim 1(A).

8. A hybridoma that secretes a monoclonal antibody according to claim 1(B).

9. A hybridoma that secretes a monoclonal antibody according to claim 1(C).

10. A hybridoma that secretes a monoclonal antibody according to claim 1(D).

11. A human Factor VIII:C monoclonal antibody produced in response to immunization with a Factor VIII:C protein composition having a Factor VIII:C protein doublet of Mᵣ = 79,000-80,000 complexed with a 92,000 protein, said composition exhibiting a biological activity of Factor VIII:C, and wherein said antibody specifically binds to at least one polypeptide derived from said composition.

12. Monoclonal antibodies prepared in response to an immunogen and specifically binding to said immunogen, said immunogen consisting of a protein composition substantially free of other proteins and consisting of a complex of a protein doublet of Mᵣ = 79,000-80,000 and a protein of Mᵣ = 92,000 wherein said doublet and said 92,000 molecular weight protein are complexed by an agent which is susceptible to chelation by EDTA, said complex exhibiting a biological activity of Factor VIII:C.

13. An immunoadsorbent column comprising particles of a solid, immunologically inert, adsorbent material to which an antibody exhibiting profile (A), (B), (C) or (D) of claim 1 or 2 is bound.

14. A column according to claim 13 wherein the adsorbent material is agarose.

## Claims (Claims for the following Contracting State(s): AT)

1. A process for the preparation of a monoclonal antibody which binds to human Factor VIII:C and exhibits one of the following binding profiles with polypeptides derived from human Factor VIII:C:
(A) binding to polypeptides derived from human Factor VIII:C which have a molecular weight of 92,000, ≥ 108,000 and 44,000 but not binding to polypeptides derived from human Factor VIII:C which -exhibit a doublet of Mᵣ = 79,000-80,000 or those which exhibit a doublet of Mᵣ = 71,000-72,000,
(B) binding to polypeptides derived from human Factor VIII:C which have a molecular weight of 92,000, ≥ 108,000 and 54,000, but not binding to polypeptides derived from human Factor VIII:C which exhibit a doublet of Mᵣ = 79,000-80,000 or those which exhibit a doublet of Mᵣ = 71,000-72,000;
(C) binding to polypeptides derived from human Factor VIII:C which exhibit a doublet of Mᵣ = 79,000-80,000 and to ≥ 108,000 polypeptides but not binding to polypeptides derived from human Factor VIII:C which have a molecular weight of 44,000, 54,000, or 92,000 and to polypeptides which exhibit a doublet of Mᵣ = 71,000-72,000;
(D) binding to polypeptides derived from human Factor VIII:C which have a molecular weight of ≥ 108,000 but not binding to polypeptides derived from human Factor VIII:C which have a molecular weight of less than 108,000;
wherein the molecular weights are determined by subjecting the polypeptides to an SDS-PAGE under reducing conditions,
which process comprises immunizing mice with a Factor VIII:C polypeptide, obtaining spleen cells from the mice and producing hybridoma cell lines therefrom according to standard techniques, culturing the hybridoma cell lines and isolating the desired monoclonal antibodies therefrom.

2. The process of claim 1 wherein the antibody is obtainable from a hybridoma formed by fusion of cells from a mouse myeloma and spleen cells from a mouse previously immunized with human Factor VIII:C, and the antibody is of the IgG class.

3. The process according to any one of claims 1 or 2 wherein the monoclonal antibody has the binding profile (B) or (C) recited therein and is further characterized in that it neutralizes the coagulant activity of human Factor VIII:C when said Factor VIII:C is bound to the antibody.

4. A process for the preaparation of a biological preparation comprising an antibody exhibiting:
(a) binding profile A of claim 1 which is essentially free from antibodies exhibiting the other three of said profiles, or
(b) binding profile B of claim 1 which is essentially free from antibodies exhibiting the other three of said profiles, or
(c) binding profile C of claim 1 which is essentially free from antibodies exhibiting the other three of said profiles; or
(d) binding profile D of claim 1 which is essentially free from antibodies exhibiting the other three of said profiles, which comprises combining the antibody with an appropriate buffer.

5. A process according to claim 4 wherein the biological preparation comprises a mixture of antibodies according to any one of claims 1 or 2 'which exhibit binding profiles A and B, which is essentially free of antibodies according to claim 1 or 2 which exhibit binding profiles C and D.

6. A process according to claim 4 wherein the biological preparation comprises a mixture of antibodies according to any one of claims 1 or 2 which exhibit binding profiles A, B, and C, which is essentially free of antibodies according to claim 1 or 2 which exhibit binding profile D.

7. A hybridoma that secretes a monoclonal antibody according to claim 1(A).

8. A hybridoma that secretes a monoclonal antibody according to claim 1(B).

9. A hybridoma that secretes a monoclonal antibody according to claim 1(C).

10. A hybridoma that secretes a monoclonal antibody according to claim 1(D).

11. A process for the preparation of a human Factor VIII:C monoclonal antibody produced in response to immunization with a Factor VIII:C protein composition having a Factor VIII:C protein doublet of Mᵣ = 79,000-80,000 complexed with a 92,000 protein, said composition exhibiting a biological activity of Factor VIII:C, and wherein said antibody specifically binds to at least one polypeptide derived from said composition which process comprises immunizing mice with said Factor VIII:C composition, obtaining spleen cells from the mice and producing hybridoma cell lines therefrom according to standard techniques, culturing the hybridoma cell lines and isolating the desired monoclonal antibodies therefrom.

12. A process for the preparation of monoclonal antibodies prepared in response to an immunogen and specifically binding to said immunogen, said immunogen consisting of a protein composition substiantially free of other proteins and consisting of a complex of a protein doublet of Mᵣ = 79,000-80,000 and a protein of Mᵣ = 92,000 wherein said doublet and said 92,000 molecular weight protein are complexed by an agent which is susceptible to chelation by EDTA, said complex exhibiting a biological activity of Factor VIII:C which process comprises immunizing mice with said immunogen, obtaining spleen cells from the mice and producing hybridoma cell lines therefrom according to standard techniques, culturing the hybridoma cell lines and isolating the desired monoclonal antibodies therefrom.

13. An immunoadsorbent column comprising particles of a solid, immunologically inert, adsorbent material to which an antibody exhibiting profile (A), (B), (C) or (D) of claim 1 or 2 is bound.

14. A column according to claim 13 wherein the adsorbent material is agarose.

## Patentansprüche (Patentansprüche für folgende(n) Vertragsstaat(en): BE, CH, LI, DE, FR, GB, IT, LU, NL, SE)

1. Monoclonaler Antikörper, der an menschlichen Faktor VIII:C bindet und eines der folgenden Bindungsprofile mit Polypeptiden zeigt, die von menschlichem Faktor VIII:C stammen:
(A) Bindung an von menschlichem Faktor VIII:C stammende Polypeptide, die ein Molekulargewicht von 92 000, ≥ 108 000 und 44 000 aufweisen, aber keine Bindung an von menschlichem Faktor VIII:C stammende Polypeptide, die eine Doppelbande mit Mᵣ = 79 000 - 80 000 zeigen, oder an solche, die eine Doppelbande mit Mᵣ = 71 000 - 72 000 zeigen;
(B) Bindung an von menschlichem Faktor VIII:C stammende Polypeptide, die ein Molekulargewicht von 92 000, ≥ 108 000 und 54 000 aufweisen, aber keine Bindung an von menschlichem Faktor VIII:C stammende Polypeptide, die eine Doppelbande mit Mᵣ = 79 000 - 80 000 zeigen, oder an solche, die eine Doppelbande mit Mᵣ = 71 000 - 72 000 zeigen;
(C) Bindung an von menschlichem Faktor VIII:C stammende Polypeptide, die eine Doppelbande mit Mᵣ = 79 000-80 000 zeigen, und an Polypeptide mit einem Molekulargewicht von ≥ 108 000, aber keine Bindung an von menschlichem Faktor VIII:C stammende Polypeptide, die ein Molekulargewicht von 44 000, 54 000 oder 92 000 aufweisen, und an Polypeptide, die eine Doppelbande mit Mᵣ = 71 000 - 72 000 zeigen;
(D) Bindung an von menschlichem Faktor VIII:C stammende Polypeptide, die ein Molekulargewicht von ≥ 108 000 aufweisen, aber keine Bindung an von menschlichem Faktor VIII:C stammende Polypeptide, die ein Molekulargewicht von weniger als 108 000 aufweisen,
wobei die Molekulargewichte dadurch bestimmt werden, daß man die Polypeptide einer SDS-PAGE unter reduzierenden Bedingungen unterwirft.

2. Monoclonaler Antikörper nach Anspruch 1, wobei der Antikörper erhältlich ist von einem Hybridom, das durch Fusion von Mausmyelomzellen und Milzzellen von einer Maus erzeugt wird, die vorher mit menschlichem Faktor VIII:C immunisiert wurde, und wobei der Antikörper zur IgG-Klasse gehört.

3. Monoclonaler Antikörper nach einem der Ansprüche 1 oder 2 mit dem darin angegebenen Bindungsprofil (B) oder (C), weiterhin dadurch gekennzeichnet, daß er die Koagulationsaktivität von menschlichem Faktor VIII:C neutralisiert, wenn der Faktor VIII:C an den Antikörper gebunden ist.

4. Biologisches Präparat, umfassend einen Antikörper, der folgende Eigenschaften besitzt:
(a) Bindungsprofil A nach Anspruch 1, im wesentlichen frei von Antikörpern, die die anderen drei Bindungsprofile zeigen, oder
(b) Bindungsprofil B nach Anspruch 1, im wesentlichen frei von Antikörpern, die die anderen drei Bindungsprofile zeigen, oder
(c) Bindungsprofil C nach Anspruch 1, im wesentlichen frei von Antikörpern, die die anderen drei Bindungsprofile zeigen, oder
(d) Bindungsprofil D nach Anspruch 1, im wesentlichen frei von Antikörpern, die die anderen drei Bindungsprofile zeigen.

5. Biologisches Präparat, umfassend ein Gemisch von Antikörpern nach einem der Ansprüche 1 oder 2, die Bindungsprofile A und B zeigen, im wesentlichen frei von Antikörpern nach Anspruch 1 oder 2, die Bindungsprofile C und D zeigen.

6. Biologisches Präparat, umfassend ein Gemisch von Antikörpern nach einem der Ansprüche 1 oder 2, die Bindungsprofile A, B und C zeigen, im wesentlichen frei von Antikörpern nach Anspruch 1 oder 2, die Bindungsprofil D zeigen.

7. Hybridom, das einen monoclonalen Antikörper nach Anspruch 1(A) sezerniert.

8. Hybridom, das einen monoclonalen Antikörper nach Anspruch 1(B) sezerniert.

9. Hybridom, das einen monoclonalen Antikörper nach Anspruch 1(C) sezerniert.

10. Hybridom, das einen monoclonalen Antikörper nach Anspruch 1(D) sezerniert.

11. Monoclonaler Antikörper gegen menschlichen Faktor VIII:C, erzeugt als Antwort auf eine Immunisierung mit einer Faktor VIII:C Protein-zusammensetzung, enthaltend ein Faktor VIII:C Protein, das eine Doppelbande mit Mᵣ = 79 000-80 000 zeigt, im Komplex mit einem 92 000-Protein, wobei die Zusammensetzung eine biologische Aktivität von Faktor VIII:C aufweist, und wobei der Antikörper spezifisch an mindestens ein aus der Zusammensetzung stammendes Polypeptid bindet.

12. Monoclonale Antikörper, die als Antwort auf ein Immunogen erzeugt werden und spezifisch an das Immunogen binden, wobei das Immunogen aus einer Proteinzusammensetzung besteht, die im wesentlichen frei von anderen Proteinen ist und aus einem Komplex eines Proteins, das eine Doppelbande von Mᵣ = 79 000 bis 80 000 zeigt, und eines Proteins mit Mᵣ = 92 000 besteht, wobei das der Doppelbande entsprechende Protein und das Protein mit dem Molekulargewicht 92 000 durch ein Mittel komplexiert sind, das empfindlich gegenüber Chelatierung durch EDTA ist, wobei der Komplex eine biologische Aktivität des Faktor VIII:C zeigt.

13. Immunoadsorbens-Säule, umfassend Teilchen eines festen, immunologisch inerten Adsorbensmaterials, an das ein Antikörper gebunden ist, der die Profile (A), (B), (C) oder (D) nach Anspruch 1 oder 2 zeigt.

14. Säule nach Anspruch 13, wobei das Adsorbensmaterial Agarose ist.

## Patentansprüche (Patentansprüche für folgende(n) Vertragsstaat(en): AT)

1. Verfahren zur Herstellung eines monoclonalen Antikörpers, der an menschlichen Faktor VIII:C bindet und eines der folgenden Bindungsprofile mit Polypeptiden zeigt, die von menschlichem Faktor VIII:C stammen:
(A) Bindung an von menschlichem Faktor VIII:C stammende Polypeptide, die ein Molekulargewicht von 92 000, ≥ 108 000 und 44 000 aufweisen, aber keine Bindung an von menschlichem Faktor VIII:C stammende Polypeptide, die eine Doppelbande mit Mᵣ = 79 000 - 80 000 zeigen, oder an solche, die eine Doppelbande mit Mᵣ = 71 000 - 72 000 zeigen;
(B) Bindung an von menschlichem Faktor VIII:C stammende Polypeptide, die ein Molekulargewicht von 92 000, ≥ 108 000 und 54 000 aufweisen, aber keine Bindung an von menschlichem Faktor VIII:C stammende Polypeptide, die eine Doppelbande mit Mᵣ = 79 000 - 80 000 zeigen, oder an solche, die eine Doppelbande mit Mᵣ = 71 000 - 72 000 zeigen;
(C) Bindung an von menschlichem Faktor VIII:C stammende Polypeptide, die eine Doppelbande mit Mᵣ = 79 000-80 000 zeigen, und an Polypeptide mit einem Molekulargewicht von ≥ 108 000, aber keine Bindung an von menschlichem Faktor VIII:C stammende Polypeptide, die ein Molekulargewicht von 44 000, 54 000 oder 92 000 aufweisen, und an Polypeptide, die eine Doppelbande mit Mᵣ = 71 000 - 72 000 zeigen;
(D) Bindung an von menschlichem Faktor VIII:C stammende Polypeptide, die ein Molekulargewicht von ≥ 108 000 aufweisen, aber keine Bindung an von menschlichem Faktor VIII:C stammende Polypeptide, die ein Molekulargewicht von weniger als 108 000 aufweisen,
wobei die Molekulargewichte dadurch bestimmt werden, daß man die Polypeptide einer SDS-PAGE unter reduzierenden Bedingungen unterwirft, wobei das Verfahren folgende Schritte umfaßt:
Immunisieren von Mäusen mit einem Faktor VIII:C-Polypeptid, Gewinnung der Milzzellen aus den Mäusen und Produktion von Hybridomzellinien daraus gemäß üblichen Verfahren, Züchtung der Hybridomzellinien und Isolierung der gewünschten monoclonalen Antikörper daraus.

2. Verfahren nach Anspruch 1, wobei der Antikörper erhältlich ist von einem Hybridom, das durch Fusion von Mausmyelomzellen und Milzzellen von einer Maus erzeugt wird, die vorher mit menschlichem Faktor VIII:C immunisiert wurde, und wobei der Antikörper zur IgG-Klasse gehört.

3. Verfahren nach einem der Ansprüche 1 oder 2, wobei der monoclonale Antikörper das darin angegebene Bindungsprofil (B) oder (C) aufweist und weiterhin dadurch gekennzeichnet ist, daß er die Koagulationsaktivität von menschlichem Faktor VIII:C neutralisiert, wenn der Faktor VIII:C an den Antikörper gebunden ist.

4. Verfahren zur Herstellung eines biologischen Präparats, umfassend einen Antikörper, der folgende Eigenschaften besitzt:
(a) Bindungsprofil A nach Anspruch 1, im wesentlichen frei von Antikörpern, die die anderen drei Bindungsprofile zeigen, oder
(b) Bindungsprofil B nach Anspruch 1, im wesentlichen frei von Antikörpern, die die anderen drei Bindungsprofile zeigen, oder
(c) Bindungsprofil C nach Anspruch 1, im wesentlichen frei von Antikörpern, die die anderen drei Bindungsprofile zeigen, oder
(d) Bindungsprofil D nach Anspruch 1, im wesentlichen frei von Antikörpern, die die anderen drei Bindungsprofile zeigen,
wobei der Antikörper mit einem geeigneten Puffer kombiniert wird.

5. Verfahren nach Anspruch 4, wobei das biologische Präparat ein Gemisch von Antikörpern nach einem der Ansprüche 1 oder 2 umfaßt, die Bindungsprofile A und B zeigen, das im wesentlichen frei ist von Antikörpern nach Anspruch 1 oder 2, die Bindungsprofile C und D zeigen.

6. Verfahren nach Anspruch 4, wobei das biologische Präparat ein Gemisch von Antikörpern nach einem der Ansprüche 1 oder 2 umfaßt, die Bindungsprofile A, B und C zeigen, das im wesentlichen frei ist von Antikörpern nach Anspruch 1 oder 2, die Bindungsprofil D zeigen.

7. Hybridom, das einen monoclonalen Antikörper nach Anspruch 1(A) sezerniert.

8. Hybridom, das einen monoclonalen Antikörper nach Anspruch 1(B) sezerniert.

9. Hybridom, das einen monoclonalen Antikörper nach Anspruch 1(C) sezerniert.

10. Hybridom, das einen monoclonalen Antikörper nach Anspruch 1(D) sezerniert.

11. Verfahren zur Herstellung eines monoclonalen Antikörpers gegen menschlichen Faktor VIII:C, erzeugt als Antwort auf eine Immunisierung mit einer Faktor VIII:C ProteinZusammensetzung, enthaltend ein Faktor VIII:C Protein, das eine Doppelbande mit Mᵣ = 79 000-80 000 zeigt, im Komplex mit einem 92 000-Protein, wobei die Zusammensetzung eine biologische Aktivität von Faktor VIII:C aufweist, und wobei der Antikörper spezifisch an mindestens ein aus der Zusammensetzung stammendes Polypeptid bindet, wobei das Verfahren folgende Schritte umfaßt:
Immunisieren von Mäusen mit einem Faktor VIII:C-Polypeptid, Gewinnung der Milzzellen aus den Mäusen und Produktion von Hybridomzellinien daraus gemäß üblichen Verfahren, Züchtung der Hybridomzellinien und Isolierung der gewünschten monoclonalen Antikörper daraus.

12. Verfahren zur Herstellung von monoclonalen Antikörpern, die als Antwort auf ein Immunogen erzeugt werden und spezifisch an das Immunogen binden, wobei das Immunogen aus einer Proteinzusammensetzung besteht, die im wesentlichen frei von anderen Proteinen ist und aus einem Komplex eines Proteins, das eine Doppelbande von Mᵣ = 79 000 bis 80 000 zeigt, und eines Proteins mit Mᵣ = 92 000 besteht, wobei das der Doppelbande entsprechende Protein und das Protein mit dem Molekulargewicht 92 000 durch ein Mittel komplexiert sind, das empfindlich gegenüber Chelatierung durch EDTA ist, wobei der Komplex eine biologische Aktivität des Faktor VIII:C zeigt, wobei das Verfahren folgende Schritte umfaßt:
Immunisieren von Mäusen mit einem Faktor VIII:C-Polypeptid, Gewinnung der Milzzellen aus den Mäusen und Produktion von Hybridomzellinien daraus gemäß üblichen Verfahren, Züchtung der Hybridomzellinien und Isolierung der gewünschten monoclonalen Antikörper daraus.

13. Immunoadsorbens-Säule, umfassend Teilchen eines festen, immunologisch inerten Adsorbensmaterials, an das ein Antikörper gebunden ist, der die Profile (A), (B), (C) oder (D) nach Anspruch 1 oder 2 zeigt.

14. Säule nach Anspruch 13, wobei das Adsorbensmaterial Agarose ist.

## Revendications (Revendications pour l'(les) Etat(s) contractant(s) suivant(s): BE, CH, LI, DE, FR, GB, IT, LU, NL, SE)

1. Anticorps monoclonal qui se lie au facteur VIII:C humain et présente un des profils de liaison suivants avec les polypeptides provenant du facteur VIII:C humain :
(A) liaison aux polypeptides provenant du facteur VIII:C humain qui possèdent une masse moléculaire de 92 000, supérieure ou égale à 108 000 et de 44 000, mais pas de liaison aux polypeptides provenant du facteur VIII:C humain qui présentent un doublet de Mᵣ = 79 000-80000 ou à ceux qui présentent un doublet de Mᵣ = 71 000-72 000 ;
(B) liaison aux polypeptides provenant du facteur VIII:C humain qui possèdent une masse moléculaire de 92 000, supérieure ou égale à 108 000 et de 54 000, mais pas de liaison aux polypeptides provenant du facteur VIII:C humain qui présentent un doublet de Mᵣ = 79 000-80 000 ou à ceux qui présentent un doublet de Mᵣ = 71 000-72 000 ;
(C) liaison aux polypeptides provenant du facteur VIII:C humain qui présentent un doublet de Mᵣ= 79 000-80000 et aux polypeptides de Mᵣ supérieure ou égale à 108 000, mais pas de liaison aux polypeptides provenant du facteur VIII:C humain qui possèdent une masse moléculaire de 44 000, de 54 000, de 92 000 et aux polypeptides qui présentent un doublet de Mᵣ = 71 000-72 000 ;
(D) liaison aux polypeptides provenant du facteur VIII:C humain qui possèdent une masse moléculaire supérieure ou égale à 108 000, mais pas de liaison aux polypeptides provenant du facteur VIII:C humain qui possèdent une masse moléculaire inférieure à 108 000 ;
les masses moléculaires étant déterminées en soumettant les polypeptides à une SDS-PAGE dans des conditions de réduction.

2. Anticorps monoclonal selon la revendication 1, lequel anticorps peut être obtenu à partir d'un hybridome formé par fusion de cellules provenant d'un myélome de souris et de cellules de rate provenant d'une souris immunisée au préalable avec le facteur VIII:C humain et lequel anticorps appartient à la classe des IgG.

3. Anticorps monoclonal selon l'une quelconque des revendications 1 et 2 ayant le profil de liaison (B) ou (C) indiqué, caractérisé de plus en ce qu'il neutralise l'activité coagulante du facteur VIII:C humain lorsque ledit facteur VIII:C est lié à l'anticorps.

4. Préparation biologique comprenant un anticorps présentant :
(a) le profil de liaison A de la revendication 1 qui est essentiellement exempt des anticorps présentant les trois autres dits profils ou
(b) le profil de liaison B de la revendication 1 qui est essentiellement exempt des anticorps présentant les trois autres dits profils ou
(c) le profil de liaison C de la revendication 1 qui est essentiellement exempt des anticorps présentant les trois autres dits profils ou
(d) le profil de liaison D de la revendication 1 qui est essentiellement exempt des anticorps présentant les trois autres dits profils.

5. Préparation biologique comprenant un mélange d'anticorps selon l'une quelconque des revendications 1 et 2 qui présentent les profils de liaison A et B, lequel est essentiellement exempt des anticorps selon la revendication 1 ou 2 qui présentent les profils de liaison C et D.

6. Préparation biologique comprenant un mélange d'anticorps selon l'une quelconque des revendications 1 et 2 qui présentent les profils de liaison A, B et C, lequel est essentiellement exempt des anticorps selon la revendication 1 ou 2 qui présentent le profil de liaison D.

7. Hybridome qui sécrète un anticorps monoclonal selon la revendication 1(A).

8. Hybridome qui sécrète un anticorps monoclonal selon la revendication 1(B).

9. Hybridome qui sécrète un anticorps monoclonal selon la revendication 1(C).

10. Hybridome qui sécrète un anticorps monoclonal selon la revendication 1(D).

11. Anticorps monoclonal dirigé contre le facteur VIII:C humain produit en réponse à l'immunisation avec une composition de protéine de facteur VIII:C ayant un doublet de protéine de facteur VIII:C de Mᵣ = 79 000-80 000 formant un complexe avec une protéine de 92000, ladite composition présentant une activité biologique de facteur VIII:C, et lequel anticorps se lie spécifiquement à au moins un polypeptide provenant de ladite composition.

12. Anticorps monoclonaux préparés en réponse à un immunogène et se liant spécifiquement audit immunogène, ledit immunogène consistant en une composition de protéine sensiblement exempte d'autres protéines et consistant en un complexe d'un doublet de protéine de Mᵣ = 79 000-80 000 et d'une protéine de Mᵣ = 92 000, où ledit doublet et ladite protéine de masse moléculaire 92 000 sont complexés par un agent qui est susceptible d'une chélation par EDTA, ledit complexe présentant une activité biologique de facteur VIII:C.

13. Colonne immunoadsorbante comprenant des particules d'un matériau adsorbant solide, immunologiquement inerte, auquel est lié un anticorps présentant le profil (A), (B), (C) ou (D) selon la revendication 1 ou 2.

14. Colonne selon la revendication 13, dans laquelle le matériau adsorbant est l'agarose.

## Revendications (Revendications pour l'(les) Etat(s) contractant(s) suivant(s): AT)

1. Procédé de préparation d'un anticorps monoclonal qui se lie au facteur VIII:C humain et présente un des profils de liaison suivants avec les polypeptides provenant du facteur VIII:C humain :
(A) liaison aux polypeptides provenant du facteur VIII:C humain qui possèdent une masse moléculaire de 92 000, supérieure ou égale à 108 000 et de 44 000, mais pas de liaison aux polypeptides provenant du facteur VIII:C humain qui présentent un doublet de Mᵣ = 79 000-80 000 ou à ceux qui présentent un doublet de Mᵣ = 71 000-72 000 ;
(B) liaison aux polypeptides provenant du facteur VIII:C humain qui possèdent une masse moléculaire de 92 000, supérieure ou égale à 108 000 et de 54 000, mais pas de liaison aux polypeptides provenant du facteur VIII:C humain qui présentent un doublet de Mᵣ= 79 000-80 000 ou à ceux qui présentent un doublet de Mᵣ = 71 000-72 000 ;
(C) liaison aux polypeptides provenant du facteur VIII:C humain qui présentent un doublet de Mᵣ= 79 000-80000 et aux polypeptides de Mᵣ supérieure ou égale à 108 000, mais pas de liaison aux polypeptides provenant du facteur VIII:C humain qui possèdent une masse moléculaire de 44 000, de 54 000, de 92 000 et aux polypeptides qui présentent un doublet de Mᵣ = 71 000-72 000 ;
(D) liaison aux polypeptides provenant du facteur VIII:C humain qui possèdent une masse moléculaire supérieure ou égale à 108 000, mais pas de liaison aux polypeptides provenant du facteur VIII:C humain qui possèdent une masse moléculaire inférieure à 108 000 ;
les masses moléculaires étant déterminées en soumettant les polypeptides à une SDS-PAGE dans des conditions de réduction,
lequel procédé comprend l'immunisation de souris avec un polypeptide de facteur VIII:C, l'obtention des cellules de rate à partir des souris et ainsi la production de lignées de cellules hybridome selon des techniques standards, la culture des lignées de cellules hybridome et ainsi l'isolement des anticorps monoclonaux souhaités.

2. Procédé selon la revendication 1, dans lequel l'anticorps peut être obtenu à partir d'un hybridome formé par fusion de cellules provenant d'un myélome de souris et de cellules de rate provenant d'une souris immunisée au préalable avec le facteur VIII:C humain et lequel anticorps appartient à la classe des IgG.

3. Procédé selon l'une quelconque des revendications 1 et 2, dans lequel l'anticorps monoclonal a le profil de liaison (B) ou (C) indiqué et est de plus caractérisé en ce qu'il neutralise l'activité coagulante du facteur VIII:C lorsque ledit facteur VIII:C est lié à l'anticorps.

4. Procédé de préparation d'une préparation biologique comprenant un anticorps présentant :
(a) le profil de liaison A de la revendication 1 qui est essentiellement exempt des anticorps présentant les trois autres dits profils ou
(b) le profil de liaison B de la revendication 1 qui est essentiellement exempt des anticorps présentant les trois autres dits profils ou
(c) le profil de liaison C de la revendication 1 qui est essentiellement exempt des anticorps présentant les trois autres dits profils ou
(d) le profil de liaison D de la revendication 1 qui est essentiellement exempt des anticorps présentant les trois autres dits profils,
lequel comprend l'association de l'anticorps avec un tampon approprié.

5. Procédé selon la revendication 4, dans lequel la préparation biologique comprend un mélange d'anticorps selon l'une quelconque des revendications 1 et 2 qui présentent les profils de liaison A et B, lequel est essentiellement exempt des anticorps selon la revendication 1 ou 2 qui présentent les profils de liaison C et D.

6. Procédé selon la revendication 4, dans lequel la préparation biologique comprend un mélange d'anticorps selon l'une quelconque des revendications 1 et 2 qui présentent les profils de liaison A, B et C, lequel est essentiellement exempt des anticorps selon la revendication 1 ou 2 qui présentent le profil de liaison D.

7. Hybridome qui sécrète un anticorps monoclonal selon la revendication 1(A).

8. Hybridome qui sécrète un anticorps monoclonal selon la revendication 1(B).

9. Hybridome qui sécrète un anticorps monoclonal selon la revendication 1(C).

10. Hybridome qui sécrète un anticorps monoclonal selon la revendication 1(D).

11. Procédé de préparation d'un anticorps monoclonal dirigé contre le facteur VIII:C humain produit en réponse à l'immunisation avec une composition de protéine de facteur VIII:C ayant un doublet de protéine de facteur VIII:C de Mᵣ = 79 000-80 000 formant un complexe avec une protéine de 92000, ladite composition présentant une activité biologique de facteur VIII:C, et lequel anticorps se lie spécifiquement à au moins un polypeptide provenant de ladite composition, lequel procédé comprend l'immunisation de souris avec ladite composition de facteur VIII:C, l'obtention des cellules de rate à partir des souris et ainsi la production de lignées de cellules hybridome selon des techniques standards, la culture des lignées de cellules hybridome et ainsi l'isolement des anticorps monoclonaux souhaités.

12. Procédé de préparation d'anticorps monoclonaux préparés en réponse à un immunogène et se liant spécifiquement audit immunogène, ledit immunogène consistant en une composition de protéine sensiblement exempte d'autres protéines et consistant en un complexe d'un doublet de protéine de Mᵣ = 79 000-80 000 et d'une protéine de Mᵣ=92 000, où ledit doublet et ladite protéine de masse moléculaire 92 000 sont complexés par un agent qui est susceptible d'une chélation par EDTA, ledit complexe présentant une activité biologique de facteur VIII:C, lequel procédé comprend l'immunisation de souris avec ledit immunogène, l'obtention des cellules de rate à partir des souris et ainsi la production de lignées de cellules hybridome selon des techniques standards, la culture des lignées de cellules hybridome et ainsi l'isolement des anticorps monoclonaux souhaités.

13. Colonne immunoadsorbante comprenant des particules d'un matériau adsorbant solide, immunologiquement inerte, auquel est lié un anticorps présentant le profil (A), (B), (C) ou (D) selon la revendication 1 ou 2.

14. Colonne selon la revendication 13, dans laquelle le matériau adsorbant est l'agarose
